(19) Europäisches Patentamt
European Patent Office
Office européen des brevets

(11) EP 3 607 601 B1

(12) **FASCICULE DE BREVET EUROPEEN**

(45) Date de publication et mention de la délivrance du brevet:
**03.05.2023 Bulletin 2023/18**

(21) Numéro de dépôt: **18718607.7**

(22) Date de dépôt: **03.04.2018**

(51) Classification Internationale des Brevets (IPC):
***H01M 10/0525*** (2010.01) ***H01M 10/0568*** (2010.01)

(52) Classification Coopérative des Brevets (CPC):
**H01M 10/0568; C07D 233/90; H01M 10/0525;**
Y02E 60/10

(86) Numéro de dépôt international:
**PCT/FR2018/050825**

(87) Numéro de publication internationale:
**WO 2018/185422 (11.10.2018 Gazette 2018/41)**

# (54) MÉLANGE DE SELS DE LITHIUM ET SES UTILISATIONS COMME ELECTROLYTE DE BATTERIE

LITHIUM-SALZ-GEMISCH UND VERWENDUNGEN DAVON ALS BATTERIEELEKTROLYT

LITHIUM SALT MIXTURE AND USES THEREOF AS A BATTERY ELECTROLYTE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.04.2017 FR 1752915**

(43) Date de publication de la demande:
**12.02.2020 Bulletin 2020/07**

(73) Titulaire: **Arkema France**
**92700 Colombes (FR)**

(72) Inventeur: **SCHMIDT, Grégory**
**69700 Saint Andeol Le Chateau (FR)**

(74) Mandataire: **Arkema Patent**
**Arkema France**
**DRD-DPI**
**420, rue d'Estienne d'Orves**
**92705 Colombes Cedex (FR)**

(56) Documents cités:
**WO-A1-2016/146925**
**FR-A1- 2 983 466**
**US-A1- 2016 126 589**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 607 601 B1

## Description

DOMAINE DE L'INVENTION

La présente demande concerne un mélange de sels de lithium, ainsi que son utilisation comme électrolyte de batterie.

ARRIERE-PLAN TECHNIQUE

Une batterie lithium-ion ou une batterie Li-Souffre comprend au moins une électrode négative (anode), une électrode positive (cathode), un séparateur et un électrolyte. L'électrolyte est généralement constitué d'un sel de lithium dissous dans un solvant qui est généralement un mélange de carbonates organiques, afin d'avoir un bon compromis entre la viscosité et la constante diélectrique. Des additifs peuvent être ensuite ajoutés pour améliorer la stabilité des sels d'électrolyte.

Parmi les sels les plus utilisés on retrouve le $LiPF_6$ (hexafluorophosphate de lithium), qui possède plusieurs des qualités requises, mais présente le désavantage de se dégrader pour former de l'acide fluorhydrique (HF) par réaction avec l'eau. Le HF formé peut entrainer une dissolution du matériau de cathode. La réaction du $LiPF_6$ avec l'eau résiduelle affecte donc la longévité de la batterie et peut engendrer des problèmes de sécurité, notamment dans le contexte de l'utilisation des batteries lithium-ion dans des véhicules de particuliers.

D'autres sels ont donc été développés, tels que le LiTFSI (bis(trifluoromethanesulfonyl)imidure de lithium) et le LiFSI (bis(fluorosulfonyl)imidure de lithium). Ces sels ne présentent que peu ou pas de décomposition spontanée, et sont plus stables vis-à-vis l'hydrolyse que $LiPF_6$. Néanmoins, le LiTFSI présente le désavantage d'être corrosif pour les collecteurs de courant, particulièrement ceux en aluminium.

FR2983466 divulgue des mélanges de sels de lithium aptes à être utilisés comme électrolytes pour batteries de type Li-ion.

Dans le domaine des batteries, il existe un besoin constant pour le développement de nouveaux sels permettant d'améliorer les performances de la batterie, telles que la durée de vie, et/ou la stabilité au cyclage, et/ou la diminution de la capacité irréversible de la batterie, les performances en puissance notamment sur une large gamme de température, tel que par exemple de -25°C environ à 60°C environ.

DESCRIPTION DE L'INVENTION

*Mélange*

La présente demande concerne un mélange de sels de lithium comprenant :

- de 85% à 99,9% molaire de bis(fluorosulfonyl)imidure de lithium (LiFSI) ; et
- de 0,1% à 15% molaire de 2-trifluorométhyl-4,5-dicyano-imidazolate de lithium (LiTDI).

Selon l'invention, les pourcentages molaires sont par rapport au nombre total de moles des composés du mélange.

Dans le cadre de l'invention, on utilise de manière équivalente les termes « sel de lithium de bis(fluorosulfonyl)imide », « lithium bis(sulfonyl)imidure », «LiFSI», «$LiN(FSO_2)_2$», « lithium de bis(sulfonyl)imide », ou « bis(fluorosulfonyl)imidure de lithium ».

Dans le cadre de l'invention, le « nombre total de moles des composés du mélange», correspond à la somme du nombre de moles de chaque composé du mélange.

Le 2-trifluorométhyl-4,5-dicyano-imidazolate de lithium, connu sous la dénomination LiTDI, possède la structure suivante :

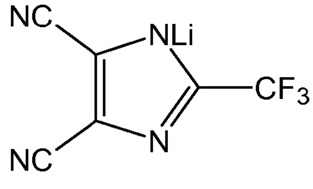

Selon un mode de réalisation, le mélange susmentionné consiste essentiellement en, de préférence est constitué de :

- de 85% à 99,9% molaire de bis(fluorosulfonyl)imidure de lithium (LiFSI) ; et
- de 0,1% à 15% molaire de 2-trifluorométhyl-4,5-dicyano-imidazolate de lithium (LiTDI).

Des impuretés peuvent être présentes dans les mélanges, à raison par exemple de moins de 3000 ppm, de préférence de moins de 1000 ppm, en particulier de moins de 500 ppm par rapport au poids total dudit mélange.
Dans le cadre de l'invention, le terme de « ppm » ou « partie par million » s'entend de ppm en poids.
Selon un mode de réalisation, le mélange selon l'invention comprend (de préférence consiste essentiellement en, et préférentiellement consiste en):

- LiFSI dans l'un des pourcentages molaires suivants : de 85% à 99%, de 85% à 98,5%, de 85% à 98%, de 85% à 97,5%, de 85% à 97%, de 85,5 à 99,9%, de 86% à 99,9%, de 86% à 99,5%, de 86,5% à 99,5%, de 87% à 99,5%, de 87% à 99%, de 87,5% à 99,9%, de 87,5% à 99,5%, de 87,5% à 99%, de 88% à 99,9%, de 88% à 99,5%, de 88% à 99%, de 89% à 99,9%, de 89% à 99,5%, de 89% à 99%, de 89,5% à 99,9%, de 89,5% à 99,5%, de 89,5% à 99%, de 90% à 99,9%, de 90% à 99,5%, de 90% à 99,5%, de 90% à 99%, de 90% à 98,5%, de 90% à 98%, de 90% à 97,5%, de 90% à 97%, de 90% à 96,5%, de 90% à 96%, de 91% à 99,9%, de 91% à 99,5%, de 91% à 99%, de 91% à 98,5%, de 91% à 98%, de 91% à 97,5%, de 91% à 97%, de 91% à 96,5%, de 91% à 96%, de 92% à 99,9%, de 92% à 99,5%, de 92% à 99%, de 92% à 98,5%, de 92% à 98%, de 92% à 97,5%, de 92% à 97%, de 92% à 96,5%, de 92% à 96%, de 93% à 99,9%, de 93% à 99,5%, de 93% à 99%, de 93% à 98,5%, de 93% à 98%, de 93% à 97,5%, de 93% à 97%, de 93% à 96,5%, de 93% à 96%, de 94% à 99,9%, de 94% à 99,5%, de 94% à 99%, de 94% à 98,5%, de 94% à 98%, de 94% à 97,5%, de 94% à 97%, de 94% à 96,5%, de 94% à 96%, de 95% à 99,9%, de 95% à 99,5%, ou de 95% à 99% ; et
- LiTDI dans l'une des pourcentages molaires suivants : de 15% à 1%, de 15% à 1,5%, de 15% à 2%, de 15% à 2,5%, de 15% à 3%, de 14,5 à 0,1%, de 14% à 0,1%, de 14% à 0,5%, de 13,5% à 0,5%, de 13% à 0,5%, de 13% à 1%, de 12,5% à 0,1%, de 12,5% à 0,5%, de 12,5% à 1%, de 12% à 0,1%, de 12% à 0,5%, de 12% à 1%, de 11% à 0,1%, de 11% à 0,5%, de 11% à 1%, de 10,5% à 0,1%, de 10,5% à 0,5%, de 11,5% à 1%, de 10% à 0,1%, de 10% à 0,5%, de 10% à 1%, de 10% à 1,5%, de 10% à 2%, de 10% à 2,5%, de 10% à 3%, de 10% à 3,5%, de 10% à 4%, de 9% à 0,1%, de 9% à 0,5%, de 9% à 1%, de 9% à 1,5%, de 9% à 2%, de 9% à 2,5%, de 9% à 3%, de 9% à 3,5%, de 9% à 4%, de 8% à 0,1%, de 9% à 0,5%, de 8% à 1%, de 8% à 1,5%, de 8% à 3%, de 8% à 2,5%, de 8% à 3%, de 8% à 3,5%, de 8% à 4%, de 7% à 0,1%, de 7% à 0,5%, de 7% à 1%, de 7% à 1,5%, de 7% à 2%, de 7% à 2,5%, de 7% à 3%, de 7% à 3,5%, de 7% à 4%, de 6% à 0,1%, de 6% à 0,5%, de 6% à 1%, de 6% à 1,5%, de 6% à 2%, de 6% à 2,5%, de 6% à 3%, de 6% à 3,5%, de 6% à 4%, de 5% à 0,1%, de 5% à 0,5%, ou de 5% à 1%.

Selon un mode de réalisation, le mélange selon l'invention comprend (de préférence consiste essentiellement en, et préférentiellement consiste en):

- LiFSI dans un pourcentage molaire supérieur ou égal à 86%, supérieur ou égal à 87%, supérieur ou égal à 88%, supérieur ou égal à 89%, supérieur ou égal à 90%, supérieur ou égal à 91%, supérieur ou égal à 92%, supérieur ou égal à 93%, supérieur ou égal à 94%, supérieur ou égal à 95%, supérieur ou égal à 96%, supérieur ou égal à 97%, supérieur ou égal à 98%, supérieur ou égal à 99%;
- LiTDI dans un pourcentage molaire inférieur ou égal à 14%, inférieur ou égal à 13%, inférieur ou égal à 12%, inférieur ou égal à 11%, inférieur ou égal à 10%, inférieur ou égal à 9%, inférieur ou égal à 8%, inférieur ou égal à 7%, inférieur ou égal à 6%, inférieur ou égal à 5%, inférieur ou égal à 4%, inférieur ou égal à 3%, inférieur ou égal à 2%, inférieur ou égal à 1%.

Selon un mode de réalisation, le mélange selon l'invention est tel que :

- le pourcentage molaire en LiFSI est supérieur ou égal à 95%; et
- le pourcentage molaire en LiTDI est inférieur ou égal à 5%.

Selon un mode de réalisation, le mélange selon l'invention comprend (de préférence consiste essentiellement en, et préférentiellement consiste en):

- de 86% à 99,9%, de préférence de 90% à 99,5%, en particulier de 92% à 98%, et préférentiellement de 93% à 97%, par exemple 95% molaire de LiFSI; et
- de 14% à 0,1%, de préférence de 10% à 0,5%, en particulier de 8% à 2%, et préférentiellement de 7% à 3%, par exemple 5% molaire de LiTDI.

La présente demande concerne aussi l'utilisation d'un mélange tel que défini ci-dessus, dans une batterie Li-ion, en particulier dans une gamme de température comprise entre -30°C et 65°C, préférentiellement entre -25°C et 60°C, de préférence à une température supérieure ou égale à 25°C, de préférence comprise entre 25°C et 65°C, avantageusement entre 40°C et 60°C. Par exemple, l'utilisation se fait dans des appareils nomades, par exemple les téléphones portables, les appareils photos, les tablettes ou les ordinateurs portables, dans des véhicules électriques, ou dans le stockage d'énergie renouvelable.

*Composition d'électrolyte*

La présente invention concerne également une composition d'électrolyte, comprenant le mélange de sels de lithium tel que défini ci-dessus, au moins un solvant, et éventuellement au moins un additif électrolytique.
De préférence, la composition ne comprend pas d'autre sel alcalin ou alcanino-terreux, que le LiFSI et LiTDI.
De préférence, la composition ne comprend pas d'autre sel de lithium que le LiFSI et le LiTDI. En particulier, la composition ne comprend pas $LiPF_6$ ni LiTFSI.
Dans le cadre de l'invention, on utilise de manière interchangeable « composition d'électrolyte », « électrolyte » et « composition électrolytique ».
Selon un mode de réalisation, la concentration molaire en LiFSI et LiTDI dans la composition d'électrolyte est inférieure ou égale à 5 mol/L, avantageusement inférieure ou égale à 4 mol/L, de préférence inférieure ou égale à 2 mol/L, préférentiellement inférieure ou égale à 1,5 mol/L, et en particulier inférieure ou égale à 1 mol/L.
Selon un mode de réalisation, les concentrations molaires en LiFSI et LiTDI dans la composition d'électrolyte sont telles que :

$$[LiFSI] + [LiTDI] \leq 1 \text{ mol/L}$$

Selon un mode de réalisation, la composition d'électrolyte susmentionnée comprend :

- de 0,85 à 0,999 mol/L de LiFSI; et
- de 0,15 à 0,001 mol/L de LiTDI.

Selon un mode de réalisation, la concentration molaire en LiFSI dans la composition d'électrolyte est choisie parmi l'une des concentrations suivantes : de 0,85 à 0,99 mol/L, de 0,85 à 0,98 mol/L, de 0,85 à 0,97 mol/L, de 0,87 à 0,99 mol/L, de 0,88 à 0,99 mol/L, de 0,89 à 0,99 mol/L, de 0,90 à 0,99 mol/L, de 0,90 à 0,98 mol/L, de 0,90 à 0,97 mol/L, de 0,90 à 0,96 mol/L, de 0,91 à 0,99 mol/L, de 0,91 à 0,98 mol/L, de 0,91 à 0,97 mol/L, de 0,91 à 0,96 mol/L, de 0,92 à 0,99 mol/L, de 0,92 à 0,98 mol/L, de 0,92 à 0,97 mol/L, de 0,92 à 0,96 mol/L, de 0,93 à 0,99 mol/L, de 0,93 à 0,98 mol/L, de 0,93 à 0,97 mol/L, de 0,93 à 0,96 mol/L, de 0,94 à 0,99 mol/L, de 0,94 à 0,98 mol/L, de 0,94 à 0,97 mol/L, de 0,94 à 0,96 mol/L, ou de 0,95 à 0,99 mol/L.
Selon un mode de réalisation, la concentration molaire en LiTDI dans la composition d'électrolyte est choisie parmi l'une des concentrations suivantes : de 0,15 à 0,01 mol/L, de 0,15 à 0,2 mol/L, de 0,15 à 0,03 mol/L, de 0,13 à 0,01 mol/L, de 0,12 à 0,01 mol/L, de 0,11 à 0,01 mol/L, de 0,10 à 0,01 mol/L, de 0,10 à 0,02 mol/L, de 0,10 à 0,03 mol/L, de 0,10 à 0,04 mol/L, de 0,09 à 0,01 mol/L, de 0,09 à 0,02 mol/L, de 0,09 à 0,03 mol/L, de 0,09 à 0,04 mol/L, de 0,08 à 0,01 mol/L, de 0,08 à 0,02 mol/L, de 0,08 à 0,03 mol/L, de 0,08 à 0,04 mol/L, de 0,07 à 0,01 mol/L, de 0,07 à 0,02 mol/L, de 0,07 à 0,03 mol/L, de 0,07 à 0,04 mol/L, de 0,06 à 0,01 mol/L, de 0,06 à 0,02 mol/L, de 0,06 à 0,03 mol/L, de 0,06 à 0,04 mol/L, ou de 0,05 à 0,01 mol/L.
Selon un mode de réalisation, la concentration molaire en LiFSI dans la composition d'électrolyte est choisie parmi l'une des concentrations suivantes : supérieure ou égale à 0,86 mol/L, supérieure ou égale à 0,87 mol/L, supérieure ou égale à 0,88 mol/L, supérieure ou égale à 0,89 mol/L, supérieure ou égale à 0,90 mol/L, supérieure ou égale à 0,91 mol/L, supérieure ou égale à 0,92 mol/L, supérieure ou égale à 0,93 mol/L, supérieure ou égale à 0,94 mol/L, supérieure ou égale à 0,95 mol/L, supérieure ou égale à 0,96 mol/L, supérieure ou égale à 0,97 mol/L, supérieure ou égale à 0,98 mol/L, ou supérieure ou égale à 0,99 mol/L.
Selon un mode de réalisation, la concentration molaire en LiTDI dans la composition d'électrolyte est choisie parmi l'une des concentrations suivantes : inférieure ou égale à 0,14 mol/L, inférieure ou égale à 0,13 mol/L, inférieure ou égale à 0,12 mol/L, inférieure ou égale à 0,11 mol/L, inférieure ou égale à 0,10 mol/L, inférieure ou égale à 0,09 mol/L, inférieure ou égale à 0,08 mol/L, inférieure ou égale à 0,07 mol/L, inférieure ou égale à 0,06 mol/L, inférieure ou égale à 0,05 mol/L, inférieure ou égale à 0,04 mol/L, inférieure ou égale à 0,03 mol/L, inférieure ou égale à 0,02 mol/L, ou inférieure ou égale à 0,01 mol/L.
Selon un mode de réalisation, la composition d'électrolyte susmentionnée est telle que

- la concentration molaire en LiFSI est supérieure ou égale à 0,95 mol/L, et

- la concentration molaire en LiTDI est inférieure ou égale à 0,05 mol/L.

Selon un mode de réalisation, la composition d'électrolyte susmentionnée comprend :

- de 0,86 à 0,999 mol/L, de préférence de 0,86 à 0,99 mol/L, notamment de 0,90 à 0,995 mol/L, en particulier de 0,92 à 0,98 mol/L, et préférentiellement de 0,93 à 0,97 mol/L, par exemple 0,95 mol/L de LiFSI; et
- de 0,14 à 0,001 mol/L, de préférence de 0,14 à 0,01 mol/L, notamment de 0,10 à 0,005 mol/L, en particulier de 0,08 à 0,2 mol/L, et préférentiellement de 0,07 à 0,03 mol/L, par exemple 0,05 mol/L de LiTDI.

Selon un mode de réalisation, la composition d'électrolyte peut comprendre un solvant ou un mélange de solvants, tel que par exemple deux, trois ou quatre solvants différents.

Le solvant de la composition d'électrolyte peut être un solvant liquide, éventuellement gélifié par un polymère, ou un solvant polymère polaire éventuellement plastifié par un liquide.

Selon un mode de réalisation, le solvant est un solvant organique, de préférence aprotique. De préférence, le solvant est un solvant organique polaire.

Selon un mode de réalisation, le solvant est choisi parmi le groupe constitué des éthers, des carbonates, des esters, des cétones, des hydrocarbures partiellement hydrogénés, des nitriles, des amides, des alcools, des sulfoxydes, du sulfolane, du nitrométhane, de la 1,3-diméthyl-2-imidazolidinone, de la 1,3-diméthyl-3,4,5,6-tétrahydro-2(1,H)-pyrimidinone, de la 3-méthyl-2-oxazolidinone, et de leurs mélanges.

Parmi les éthers, on peut citer les éthers linéaires ou cycliques, tels que par exemple le diméthoxyéthane (DME), les éthers méthyliques des oligoéthylène glycols de 2 à 5 unités oxyéthylènes, le dioxolane, le dioxane, le dibutyle éther, le tétrahydrofurane, et leurs mélanges.

Parmi les esters, on peut citer les esters d'acide phosphorique ou les esters de sulfite. On peut par exemple citer le formate de méthyle, l'acétate de méthyle, le propionate de méthyle, l'acétate d'éthyle, l'acétate de butyle, la gamma butyrolactone ou leurs mélanges.

Parmi les cétones, on peut notamment citer la cyclohexanone.

Parmi les alcools, on peut par exemple citer l'alcool éthylique, l'alcool isopropylique.

Parmi les nitriles, on peut citer par exemple l'acétonitrile, le pyruvonitrile, le propionitrile, le méthoxypropionitrile, le diméthylaminopropionitrile, le butyronitrile, l'isobutyronitrile, le valéronitrile, le pivalonitrile, l'isovaléronitrile, le glutaronitrile, le méthoxyglutaronitrile, le 2-méthylglutaronitrile, le 3-méthylglutaronitrile, l'adiponitrile, le malononitrile, et leurs mélanges.

Parmi les carbonates, on peut citer par exemple les carbonates cycliques tels que par exemple le carbonate d'éthylène (EC), le carbonate de propylène (PC), le carbonate de butylène (BC), le carbonate de diméthyle (DMC), le carbonate de diéthyle (DEC), le carbonate de méthyle éthyle (EMC) (CAS N° 623-53-0), le carbonate de diphényle, le carbonate de méthyle phényle, le carbonate de dipropyle (DPC), le carbonate de méthyle et de propyle (MPC), le carbonate d'éthyle et de propyle (EPC), le carbonate de vinylène (VC), le fluoroethylène carbonate (FEC), le trifluoropropylène carbonate ou leurs mélanges.

Le solvant particulièrement préféré est choisi parmi les carbonates et leurs mélanges. On peut notamment citer les mélanges suivants :

- carbonate d'éthylène (EC)/carbonate de propylène (PC)/carbonate de diméthyle (DMC) dans un rapport massique 1/1/1 ;
- carbonate d'éthylène (EC)/carbonate de propylène (PC)/carbonate de diéthyle (DEC) dans un rapport massique 1/1/1 ;
- carbonate d'éthylène (EC)/carbonate de propylène (PC)/carbonate de méthyle éthyle (EMC) dans un rapport massique 1/1/1 ;
- carbonate d'éthylène (EC)/carbonate de diméthyle (DMC) dans un rapport massique 1/1 ;
- carbonate d'éthylène (EC)/carbonate de diéthyle (DEC) dans un rapport massique1/1 ;
- carbonate d'éthylène (EC)/carbonate de méthyle éthyle (EMC) dans un rapport massique 1/1 ;
- carbonate d'éthylène (EC)/carbonate de diméthyle (DMC) dans un rapport massique dans un rapport volumique 3/7 ;
- carbonate d'éthylène (EC)/carbonate de diéthyle (DEC) dans un rapport volumique 3/7 ;
- carbonate d'éthylène (EC)/carbonate de méthyle éthyle (EMC) dans un rapport volumique 3/7.

Selon un mode de réalisation, la composition d'électrolyte peut comprendre au moins un additif électrolytique.

De préférence, l'additif électrolytique est choisi parmi le groupe constitué du carbonate de fluoroéthylène (FEC), carbonate de vinylène, 4-vinyl-1,3-dioxolan-2-one, la pyridazine, la vinyl pyridazine, la quinoline, la vinyl quinoline, le butadiène, le sebaconitrile, le LiB(C2O4)2, le nitrate de lithium, les alkyldisulfure, le fluorotoluène, le 1,4-diméthoxytétrafluorotoluène, les oximes, les époxydes aliphatiques, les biphényls halogénés, les acides metacryliques, le carbonate

d'allyle éthyle, acétate de vinyle, adipate de divinyle, acrylonitrile, 2-vinylpyridine, anhydride maléïque, cinnamate de méthyle, phosphonates, composés silane contenant un vinyle, 2-cyanofurane et de leurs mélanges, l'additif électrolytique étant de préférence le carbonate de fluoroéthylène (FEC).

Par exemple, la teneur en additif électrolytique dans la composition d'électrolyte est comprise entre 0,01% et 10%, de préférence entre 0,1% et 4% en masse par rapport à la masse totale de la composition. En particulier, la teneur en additif électrolytique dans la composition d'électrolyte est inférieure ou égale à 2% en masse par rapport à la masse totale de la composition.

Selon un mode de réalisation, la composition d'électrolyte selon l'invention est choisie parmi l'une des compositions suivantes :

- i) 0,85 mol/L de LiFSI et 0,15 mol/L de LiTDI, carbonate de fluoroéthylène comme additif électrolytique (en particulier à une teneur inférieure ou égale à 2% massique), mélange de EC/EMC dans un rapport 3/7 en volume comme solvant;
- ii) 0,90 mol/L de LiFSI et 0,10 mol/L de LiTDI, carbonate de fluoroéthylène comme additif électrolytique (en particulier à une teneur inférieure ou égale à 2% massique), mélange de EC/EMC dans un rapport 3/7 en volume comme solvant;
- iii) 0,95 mol/L de LiFSI et 0,05 mol/L de LiTDI, carbonate de fluoroéthylène comme additif électrolytique (en particulier à une teneur inférieure ou égale à 2% massique), mélange de EC/EMC dans un rapport 3/7 en volume comme solvant;
- iii) 0,96 mol/L de LiFSI et 0,04 mol/L de LiTDI, carbonate de fluoroéthylène comme additif électrolytique (en particulier à une teneur inférieure ou égale à 2% massique), mélange de EC/EMC dans un rapport 3/7 en volume comme solvant;
- iv) 0,97 mol/L de LiFSI et 0,03 mol/L de LiTDI, carbonate de fluoroéthylène comme additif électrolytique (en particulier à une teneur inférieure ou égale à 2% massique), mélange de EC/EMC dans un rapport 3/7 en volume comme solvant;
- v) 0,98 mol/L de LiFSI et 0,02 mol/L de LiTDI, carbonate de fluoroéthylène comme additif électrolytique (en particulier à une teneur inférieure ou égale à 2% massique), mélange de EC/EMC dans un rapport 3/7 en volume comme solvant;
- vi) 0,99 mol/L de LiFSI et 0,01 mol/L de LiTDI, carbonate de fluoroéthylène comme additif électrolytique (en particulier à une teneur inférieure ou égale à 2% massique), mélange de EC/EMC dans un rapport 3/7 en volume comme solvant.

La composition, de préférence la composition d'électrolyte, peut être préparée par dissolution, de préférence sous agitation, des sels dans des proportions appropriées de solvant(s).

La présente demande concerne aussi l'utilisation d'une composition d'électrolyte telle que définie ci-dessus, dans une batterie Li-ion, en particulier dans une gamme de température comprise entre -30°C et 65°C, préférentiellement entre -25°C et 60°C, de préférence à une température supérieure ou égale à 25°C, de préférence comprise entre 25°C et 65°C, avantageusement entre 40°C et 60°C. Par exemple, l'utilisation se fait dans des appareils nomades, par exemple les téléphones portables, les appareils photos, les tablettes ou les ordinateurs portables, dans des véhicules électriques, ou dans le stockage d'énergie renouvelable.

*Cellule électrochimique*

La présente demande concerne également une cellule électrochimique comportant une électrode négative, une électrode positive, et une composition d'électrolyte telle qu'ici définie ci-dessus, interposée entre l'électrode négative et l'électrode positive. La cellule électrochimique peut aussi comprendre un séparateur, dans lequel est imprégnée la composition d'électrolyte telle que définie ci-dessus.

La présente invention concerne également une batterie comprenant au moins une cellule électrochimique telle que décrite ci-dessus. Lorsque la batterie comprend plusieurs cellules électrochimiques selon l'invention, lesdites cellules peuvent être assemblées en série et/ou en parallèle.

Dans le cadre de l'invention, par électrode négative, on entend l'électrode qui fait office d'anode, quand la batterie débite du courant (c'est-à-dire lorsqu'elle est en processus de décharge) et qui fait office de cathode lorsque la batterie est en processus de charge.

L'électrode négative comprend typiquement un matériau électrochimiquement actif, éventuellement un matériau conducteur électronique, et éventuellement un liant.

Dans le cadre de l'invention, on entend par « matériau électrochimiquement actif », un matériau capable d'insérer de manière réversible des ions.

Dans le cadre de l'invention, on entend par « matériau conducteur électronique » un matériau capable conduire les électrons.

Selon un mode de réalisation, l'électrode négative de la cellule électrochimique comprend, comme matériau électrochimiquement actif, du graphite, du lithium, un alliage de lithium, un titanate de lithium de type $Li_4Ti_5O_{12}$ ou $TiO_2$, du silicium ou un alliage de lithium et silicium, un oxyde d'étain, un composé intermétallique de lithium, ou un de leurs mélanges.

L'électrode négative peut comprendre du lithium, celui-ci peut alors être constitué d'un film de lithium métallique ou d'un alliage comprenant du lithium. Un exemple d'électrode négative peut comprendre un film de lithium vif préparé

par laminage, entre des rouleaux, d'un feuillard de lithium.

Dans le cadre de l'invention, par électrode positive, on entend l'électrode qui fait office de cathode, quand la batterie débite du courant (c'est-à-dire lorsqu'elle est en processus de décharge) et qui fait office d'anode lorsque la batterie est en processus de charge.

L'électrode positive comprend typiquement un matériau électrochimiquement actif, éventuellement un matériau conducteur électronique, et éventuellement un liant.

Dans un autre mode de réalisation, l'électrode positive de la cellule électrochimique comprend un matériau électrochimiquement actif choisi parmi le dioxyde de manganèse ($MnO_2$), l'oxyde de fer, l'oxyde de cuivre, l'oxyde de nickel, les oxydes composites lithium-manganèse (par exemple $Li_xMn_2O4$ ou $Li_xMnO_2$), les oxydes compositions lithium-nickel (par exemple $Li_xNiO_2$), les oxydes compositions lithium-cobalt (par exemple $Li_xCoO_2$), les oxydes composites lithium-nickel-cobalt (par exemple $LiNi_{1-y}Co_yO_2$), les oxydes composites lithium-nickel-cobalt-manganèse (par exemple $LiNi_xMn_yCo_zO_2$ avec x+y+z = 1), les oxydes composites lithium-nickel-cobalt-manganèse enrichis en lithium (par exemple $Li_{1+x}(NiMnCo)_{1-x}O_2$), les oxydes composites de lithium et de métal de transition, les oxydes composites de lithium-manganèse-nickel de structure spinelle (par exemple $Li_xMn_{2-y}Ni_yO_4$), les oxydes de lithium-phosphore de structure olivine (par exemple $Li_xFePO_4$, $Li_xFe_{1-y}Mn_yPO_4$ ou $Li_xCoPO_4$), le sulfate de fer, les oxydes de vanadium, et leurs mélanges.

De préférence, l'électrode positive est comprend un matériau électrochimiquement actif choisi parmi $LiCoO_2$, $LiFePO_4$ (LFP), $LiMn_xCo_yNi_zO_2$(NMC, avec x+y+z = 1), $LiFePO_4F$, $LiFeSO_4F$, $LiNiCoAlO_2$ et leurs mélanges.

Le matériau de l'électrode positive peut aussi comprendre, outre le matériau électrochimiquement actif, un matériau conducteur électronique comme une source de carbone, incluant, par exemple, du noir de carbone, du carbone Ketjen®, du carbone Shawinigan, du graphite, du graphène, des nanotubes de carbone, des fibres de carbone (tels les fibres de carbone formées en phase gazeuse (VGCF), du carbone non-poudreux obtenu par carbonisation d'un précurseur organique, ou une combinaison de deux ou plus de ceux-ci. D'autres additifs peuvent aussi être présents dans le matériau de l'électrode positive, comme des sels de lithium ou des particules inorganiques de type céramique ou verre, ou encore d'autres matériaux actifs compatibles (par exemple, du soufre).

Le matériau de l'électrode positive peut aussi comprendre un liant. Des exemples non-limitatifs de liants comprennent les liants polymères polyéthers linéaires, ramifiés et/ou réticulé (par exemple, des polymères basés sur le poly(oxyde d'éthylène) (PEO), ou le poly(oxyde de propylène) (PPO) ou d'un mélange des deux (ou un co-polymère EO/PO), et comprenant éventuellement des unités réticulables), des liants solubles dans l'eau (tels que SBR (caoutchouc styrène-butadiène), NBR (caoutchouc acrylonitrile-butadiène), HNBR (NBR hydrogéné), CHR (caoutchouc d'épichlorohydrine), ACM (caoutchouc d'acrylate)), ou des liants de type polymères fluorés (tels que PVDF (fluorure de polyvinylidène), PTFE (polytétrafluoroéthylène), et leurs combinaisons. Certains liants, comme ceux solubles dans l'eau, peuvent aussi comprendre un additif comme le CMC (carboxyméthylcellulose).

Le mélange de sels selon l'invention a avantageusement une bonne conductivité ionique en solution. De plus, le mélange de sels selon l'invention permet avantageusement d'améliorer les performances en puissance de la batterie, ce qui permet par exemple de recharger plus vite la batterie, ou encore de fournir la puissance nécessaire en cas de pic d'énergie.

Le mélange de sels selon l'invention permet également avantageusement d'avoir de bonnes performances, notamment en termes de puissance, sur une large gamme de température, par exemple à froid, ou sur une gamme de température allant d'environ -25°C à environ 60°C.

Dans le cadre de l'invention, par « comprise entre x et y » ou « entre x et y », on entend un intervalle dans lequel les bornes x et y sont incluses. Par exemple, la gamme «comprise entre 85% et 99,9% » ou « allant de 85% à 99,9% » inclus notamment les valeurs 85 et 99,9%.

Tous les modes de réalisation décrits ci-dessus peuvent être combinés les uns avec les autres.

Les exemples suivants illustrent l'invention sans toutefois la limiter.

## PARTIE EXPERIMENTALE

Abréviations

EC : carbonate d'éthylène
EMC : carbonate de méthyle éthyle
FEC : carbonate de fluoroéthylène

Fournisseurs

EC : BASF Corporation
EMC : BASF Corporation
FEC : BASF Corporation

Le LiFSI utilisé est notamment obtenu par le procédé décrit dans la demande WO2015/158979, tandis que le LiTDI est issu du procédé décrit dans la demande WO2013/072591.

**Exemple 1 : Conductivité ionique mesurée par spectroscopie d'impédance**

Deux électrolytes ont été préparés selon les compositions suivantes :

- composition 1 (selon l'invention): 0.95M LiFSI, 0.05M LiTDI, mélange de solvants EC/EMC (carbonate d'éthylène/carbonate de méthyle éthyle) 3/7 (ratio en volume), 2% en poids en FEC (par rapport au poids total de la composition);
- composition 2 (comparative) : 0.80M LiFSI, 0.20M LiTDI, mélange de solvants EC/EMC 3/7 (carbonate d'éthylène/carbonate de méthyle éthyle) (ratio en volume), 2% en poids en FEC (par rapport au poids total de la composition).

Une cellule de conductivité est alors plongée dans chacune des solutions et trois spectroscopies d'impédance ont été réalisées. Ces spectroscopies sont réalisées entre 500 mHz et 100 kHz avec une amplitude de 10 mV. La constante de la cellule utilisée est de 1.12 et la conductivité ionique est calculée selon la formule suivante :

$$\sigma = \frac{1}{R} \times 1.12$$

avec R représente la résistance qui est obtenue par régression linéaire de la courbe Im(Z) = f (Re(Z)). Dans le cas particulier de Im(Z) = 0, R est égale à l'opposé de l'ordonnée à l'origine divisée par le coefficient directeur de l'équation de la régression linéaire.

| Composition | Conductivité (mS/cm) | R1 | R2 | R3 | R moyen |
|---|---|---|---|---|---|
| 1 | 14.66 | 75.72 | 76.4 | 77.13 | 76.417 |
| 2 | 13.50 | 82.56 | 83.4 | 83 | 82.987 |

Le mélange 0.95M LiFSI / 0.05M LiTDI présente avantageusement une meilleure conductivité ionique que le mélange 0.8 LiFSI / 0.2 LiTDI.

**Exemple 2 : Test de puissance**

Un test de Ragone Plot a été réalisé avec les compositions 1 et 2 suivantes :

- composition 1 (selon l'invention): 0.95M LiFSI, 0.05M LiTDI, mélange de solvants EC/EMC (carbonate d'éthylène/carbonate de méthyle éthyle) 3/7 (ratio en volume), 2% en poids en FEC (par rapport au poids total de la composition);
- composition 2 (comparative) : 0.80M LiFSI, 0.20M LiTDI, mélange de solvants EC/EMC (carbonate d'éthylène/carbonate de méthyle éthyle) 3/7 (ratio en volume), 2% en poids en FEC (par rapport au poids total de la composition).

Méthode : la méthode consiste à augmenter la vitesse de décharge d'une batterie afin d'observer la capacité de l'électrolyte de pouvoir répondre au stress imposé par le circuit électrique.

Système utilisé :

Cathode : $LiNi_{0.33}Mn_{0.33}Co_{0.33}O_2$ (89%), Fibre de carbone VGCF (2.5%), du noir de carbone (2.5%) et 6% de liant Pvdf.
Anode : Lithium métal

Le courant a été varié entre 2.7 et 4.2 V, avec les décharges opérées dans l'ordre suivant : C/20, C/10, C/5, C/2, C et 2C.

Deux cycles de formation à C/20 sont réalisés avant l'étude pour former toutes les couches de passivation.

Résultats :

Les résultats observés sont les suivants :

| Composition 1 (invention) | | |
|---|---|---|
| | Décharge | Ragone |
| 1 | C/20 | 100 |
| 2 | C/10 | 103,673781 |
| 3 | C/5 | 104,59516 |
| 4 | C/2 | 103,072243 |
| 5 | C | 99,3299947 |
| 6 | 2C | 92,1065553 |

| Composition 2 (comparative) | | |
|---|---|---|
| | Décharge | Ragone |
| 1 | C/20 | 100 |
| 2 | C/10 | 102,160088 |
| 3 | C/5 | 100,057551 |
| 4 | C/2 | 94,490528 |
| 5 | C | 89,4708167 |
| 6 | 2C | 84,5363772 |

Les résultats montrent que la composition 1 permet de travailler à des régimes de puissance plus élevés que la composition 2. Ces régimes élevés sont particulièrement recherchés dans les batteries commerciales dans le cadre des appareils nomades qui demandent toujours plus de puissance, et des véhicules électriques qui du fait de leur faible autonomie demandent des recharges rapides et donc des électrolytes permettant de travailler à régimes élevés.

## Revendications

1. Mélange de sels de lithium comprenant :

   - de 85% à 99,9% molaire de bis(fluorosulfonyl)imidure de lithium ; et
   - de 0,1% à 15% molaire de 2-trifluorométhyl-4,5-dicyano-imidazolate de lithium.

2. Mélange selon la revendication 1 comprenant :

   - le bis(fluorosulfonyl)imidure de lithium dans l'un des pourcentages molaires suivants : de 85% à 99%, de 85% à 98,5%, de 85% à 98%, de 85% à 97,5%, de 85% à 97%, de 85,5 à 99,9%, de 86% à 99,9%, de 86% à 99,5%, de 86,5% à 99,5%, de 87% à 99,5%, de 87% à 99%, de 87,5% à 99,9%, de 87,5% à 99,5%, de 87,5% à 99%, de 88% à 99,9%, de 88% à 99,5%, de 88% à 99%, de 89% à 99,9%, de 89% à 99,5%, de 89% à 99%, de

89,5% à 99,9%, de 89,5% à 99,5%, de 89,5% à 99%, de 90% à 99,9%, de 90% à 99,5%, de 90% à 99,5%, de 90% à 99%, de 90% à 98,5%, de 90% à 98%, de 90% à 97,5%, de 90% à 97%, de 90% à 96,5%, de 90% à 96%, de 91% à 99,9%, de 91% à 99,5%, de 91% à 99%, de 91% à 98,5%, de 91% à 98%, de 91% à 97,5%, de 91% à 97%, de 91% à 96,5%, de 91% à 96%, de 92% à 99,9%, de 92% à 99,5%, de 92% à 99%, de 92% à 98,5%, de 92% à 98%, de 92% à 97,5%, de 92% à 97%, de 92% à 96,5%, de 92% à 96%, de 93% à 99,9%, de 93% à 99,5%, de 93% à 99%, de 93% à 98,5%, de 93% à 98%, de 93% à 97,5%, de 93% à 97%, de 93% à 96,5%, de 93% à 96%, de 94% à 99,9%, de 94% à 99,5%, de 94% à 99%, de 94% à 98,5%, de 94% à 98%, de 94% à 97,5%, de 94% à 97%, de 94% à 96,5%, de 94% à 96%, de 95% à 99,9%, de 95% à 99,5%, ou de 95% à 99% ; et

- le 2-trifluorométhyl-4,5-dicyano-imidazolate de lithium dans l'un des pourcentages molaires suivants: de 15% à 1%, de 15% à 1,5%, de 15% à 2%, de 15% à 2,5%, de 15% à 3%, de 14,5 à 0,1%, de 14% à 0,1%, de 14% à 0,5%, de 13,5% à 0,5%, de 13% à 0,5%, de 13% à 1%, de 12,5% à 0,1%, de 12,5% à 0,5%, de 12,5% à 1%, de 12% à 0,1%, de 12% à 0,5%, de 12% à 1%, de 11% à 0,1%, de 11% à 0,5%, de 11% à 1%, de 10,5% à 0,1%, de 10,5% à 0,5%, de 11,5% à 1%, de 10% à 0,1%, de 10% à 0,5%, de 10% à 1%, de 10% à 1,5%, de 10% à 2%, de 10% à 2,5%, de 10% à 3%, de 10% à 3,5%, de 10% à 4%, de 9% à 0,1%, de 9% à 0,5%, de 9% à 1%, de 9% à 1,5%, de 9% à 2%, de 9% à 2,5%, de 9% à 3%, de 9% à 3,5%, de 9% à 4%, de 8% à 0,1%, de 9% à 0,5%, de 8% à 1%, de 8% à 1,5%, de 8% à 3%, de 8% à 2,5%, de 8% à 3%, de 8% à 3,5%, de 8% à 4%, de 7% à 0,1%, de 7% à 0,5%, de 7% à 1%, de 7% à 1,5%, de 7% à 2%, de 7% à 2,5%, de 7% à 3%, de 7% à 3,5%, de 7% à 4%, de 6% à 0,1%, de 6% à 0,5%, de 6% à 1%, de 6% à 1,5%, de 6% à 2%, de 6% à 2,5%, de 6% à 3%, de 6% à 3,5%, de 6% à 4%, de 5% à 0,1%, de 5% à 0,5%, ou de 5% à 1%.

**3.** Mélange selon l'une quelconque des revendications 1 ou 2, dans lequel :

- le pourcentage molaire en bis(fluorosulfonyl)imidure de lithium est supérieur ou égal à 95%; et
- le pourcentage molaire en 2-trifluorométhyl-4,5-dicyano-imidazolate de lithium est inférieur ou égal à 5%.

**4.** Mélange selon l'une quelconque des revendications 1 ou 2, comprenant :

- de 86% à 99,9%, de préférence de 90% à 99,5%, en particulier de 92% à 98%, et préférentiellement de 93% à 97%, par exemple 95% molaire de bis(fluorosulfonyl)imidure de lithium; et
- de 14% à 0,1%, de préférence de 10% à 0,5%, en particulier de 8% à 2%, et préférentiellement de 7% à 3%, par exemple 5% molaire de 2-trifluorométhyl-4,5-dicyano-imidazolate de lithium.

**5.** Utilisation d'un mélange selon l'une quelconque des revendications 1 à 4, dans une batterie Li-ion, en particulier dans une gamme de température comprise entre - 30°C et 65°C, préférentiellement entre -25°C et 60°C, de préférence à une température supérieure ou égale à 25°C, de préférence comprise entre 25°C et 65°C, avantageusement entre 40°C et 60°C.

**6.** Composition d'électrolyte, comprenant le mélange de sels de lithium selon l'une quelconque des revendications 1 à 4, au moins un solvant, et éventuellement au moins un additif électrolytique.

**7.** Composition selon la revendication 6, dans laquelle la concentration molaire en bis(fluorosulfonyl)imidure de lithium et 2-trifluorométhyl-4,5-dicyano-imidazolate de lithium dans la composition d'électrolyte est inférieure ou égale à 5 mol/L, avantageusement inférieure ou égale à 4 mol/L, de préférence inférieure ou égale à 2 mol/L, préférentiellement inférieure ou égale à 1,5 mol/L, et en particulier inférieure ou égale à 1 mol/L.

**8.** Composition selon l'une quelconque des revendications 6 ou 7, comprenant :

- de 0,85 à 0,999 mol/L de bis(fluorosulfonyl)imidure de lithium ; et
- de 0,15 à 0,001 mol/L de 2-trifluorométhyl-4,5-dicyano-imidazolate de lithium.

**9.** Composition selon l'une quelconque des revendications 6 à 8, comprenant :

- de 0,86 à 0,999 mol/L, de préférence de 0,86 à 0,99 mol/L, notamment de 0,90 à 0,995 mol/L, en particulier de 0,92 à 0,98 mol/L, et préférentiellement de 0,93 à 0,97 mol/L, par exemple 0,95 mol/L de bis(fluorosulfonyl)imidure de lithium ; et
- de 0,14 à 0,001 mol/L, de préférence de 0,14 à 0,01 mol/L, notamment de 0,10 à 0,005 mol/L, en particulier de 0,08 à 0,2 mol/L, et préférentiellement de 0,07 à 0,03 mol/L, par exemple 0,05 mol/L de 2-trifluorométhyl-4,5-dicyano-imidazolate de lithium.

**10.** Composition selon l'une quelconque des revendications 6 à 9, dans lequel le solvant est choisi parmi le groupe constitué des éthers, des carbonates, des esters, des cétones, des hydrocarbures partiellement hydrogénés, des nitriles, des amides, des alcools, des sulfoxydes, du sulfolane, du nitrométhane, de la 1,3-diméthyl-2-imidazolidinone, de la 1,3-diméthyl-3,4,5,6-tétrahydro-2(1,H)-pyrimidinone, de la 3-méthyl-2-oxazolidinone, et de leurs mélanges.

**11.** Composition selon l'une quelconque des revendications 6 à 10, dans lequel le solvant est choisi parmi les carbonates et leurs mélanges, par exemple parmi les mélanges suivants :

- carbonate d'éthylène/carbonate de propylène/carbonate de diméthyle dans un rapport massique 1/1/1 ;
- carbonate d'éthylène/carbonate de propylène/carbonate de diéthyle dans un rapport massique 1/1/1 ;
- carbonate d'éthylène/carbonate de propylène/carbonate de méthyle éthyle dans un rapport massique 1/1/1 ;
- carbonate d'éthylène/carbonate de diméthyle dans un rapport massique 1/1 ;
- carbonate d'éthylène/carbonate de diéthyle dans un rapport massique 1/1 ;
- carbonate d'éthylène/carbonate de méthyle éthyle dans un rapport massique 1/1 ;
- carbonate d'éthylène/carbonate de diméthyle dans un rapport volumique 3/7 ;
- carbonate d'éthylène/ carbonate de diéthyle dans un rapport volumique 3/7 ;
- carbonate d'éthylène/carbonate de méthyle éthyle dans un rapport volumique 3/7.

**12.** Composition selon l'une quelconque des revendications 6 à 11, dans lequel l'additif électrolytique est choisi parmi le groupe constitué du carbonate de fluoroéthylène, carbonate de vinylène, 4-vinyl-1,3-dioxolan-2-one, la pyridazine, la vinyl pyridazine, la quinoline, la vinyl quinoline, le butadiène, le sebaconitrile, le $LiB(C_2O4)_2$, le nitrate de lithium, les alkyldisulfure, le fluorotoluène, le 1,4-diméthoxytétrafluorotoluène, les oximes, les époxydes aliphatiques, les biphényls halogénés, les acides metacryliques, le carbonate d'allyle éthyle, acétate de vinyle, adipate de divinyle, acrylonitrile, 2-vinylpyridine, anhydride maléïque, cinnamate de méthyle, phosphonates, composés silane contenant un vinyle, 2-cyanofurane et de leurs mélanges, l'additif électrolytique étant de préférence le carbonate de fluoroéthylène.

**13.** Utilisation d'une composition d'électrolyte selon l'une quelconque des revendications 6 à 12, dans une batterie Li-ion, en particulier dans une gamme de température comprise entre -30°C et 65°C, préférentiellement entre -25°C et 60°C, de préférence à une température supérieure ou égale à 25°C, de préférence comprise entre 25°C et 65°C, avantageusement entre 40°C et 60°C.

**14.** Cellule électrochimique comportant une électrode négative, une électrode positive, et une composition d'électrolyte selon l'une quelconque des revendications 6 à 12, interposée entre l'électrode négative et l'électrode positive.

**15.** Batterie comprenant au moins une cellule électrochimique selon la revendication 14.

## Patentansprüche

**1.** Mischung von Lithiumsalzen, umfassend:

- 85 bis 99,9 Mol-% Lithiumbis(fluorsulfonyl)imid und
- 0,1 bis 15 Mol-% Lithium-2-trifluormethyl-4,5-dicyanoimidazolat.

**2.** Mischung nach Anspruch 1, umfassend:

- Lithiumbis(fluorsulfonyl)imid in einem der folgenden Molprozentanteile: von 85% bis 99%, von 85% bis 98,5%, von 85% bis 98%, von 85% bis 97,5%, von 85% bis 97%, von 85,5% bis 99,9%, von 86% bis 99,9%, von 86% bis 99,5%, von 86,5% bis 99,5%, von 87% bis 99,5%, von 87% bis 99%, von 87,5% bis 99,9%, von 87,5% bis 99,5%, von 87,5% bis 99%, von 88% bis 99,9%, von 88% bis 99,5%, von 88% bis 99%, von 89% bis 99,9%, von 89% bis 99,5%, von 89% bis 99%, von 89,5% bis 99,9%, von 89,5% bis 99,5%, von 89,5% bis 99%, von 90% bis 99,9%, von 90% bis 99,5%, von 90% bis 99,5%, von 90% bis 99%, von 90% bis 98,5%, von 90% bis 98%, von 90% bis 97,5%, von 90% bis 97%, von 90% bis 96,5%, von 90% bis 96%, von 91% bis 99,9%, von 91% bis 99,5%, von 91% bis 99%, von 91% bis 98,5%, von 91% bis 98%, von 91% bis 97,5%, von 91% bis 97%, von 91% bis 96,5%, von 91% bis 96%, von 92% bis 99,9%, von 92% bis 99,5%, von 92% bis 99%, von

92% bis 98,5%, von 92% bis 98%, von 92% bis 97,5%, von 92% bis 97%, von 92% bis 96,5%, von 92% bis 96%, von 93% bis 99,9%, von 93% bis 99,5%, von 93% bis 99%, von 93% bis 98,5%, von 93% bis 98%, von 93% bis 97,5%, von 93% bis 97%, von 93% bis 96,5%, von 93% bis 96%, von 94% bis 99,9%, von 94% bis 99,5%, von 94% bis 99%, von 94% bis 98,5%, von 94% bis 98%, von 94% bis 97,5%, von 94% bis 97%, von 94% bis 96,5%, von 94% bis 96%, von 95% bis 99,9%, von 95% bis 99,5% oder von 95% bis 99%; und

- Lithium-2-trifluormethyl-4,5-dicyanoimidazolat in einem der folgenden Molprozentanteile: von 15% bis 1%, von 15% bis 1,5%, von 15% bis 2%, von 15% bis 2,5%, von 15% bis 3%, von 14,5% bis 0,1%, von 14% bis 0,1%, von 14% bis 0,5%, von 13,5% bis 0,5%, von 13% bis 0,5%, von 13% bis 1%, von 12,5% bis 0,1%, von 12,5% bis 0,5%, von 12,5% bis 1%, von 12% bis 0,1%, von 12% bis 0,5%, von 12% bis 1%, von 11% bis 0,1%, von 11% bis 0,5%, von 11% bis 1%, von 10,5% bis 0,1%, von 10,5% bis 0,5%, von 11,5% bis 1%, von 10% bis 0,1%, von 10% bis 0,5%, von 10% bis 1%, von 10% bis 1,5%, von 10% bis 2%, von 10% bis 2,5%, von 10% bis 3%, von 10% bis 3,5%, von 10% bis 4%, von 9% bis 0,1%, von 9% bis 0,5%, von 9% bis 1%, von 9% bis 1,5%, von 9% bis 2%, von 9% bis 2,5%, von 9% bis 3%, von 9% bis 3,5%, von 9% bis 4%, von 8% bis 0,1%, von 9% bis 0,5%, von 8% bis 1%, von 8% bis 1,5%, von 8% bis 3%, von 8% bis 2,5%, von 8% bis 3%, von 8% bis 3,5%, von 8% bis 4%, von 7% bis 0,1%, von 7% bis 0,5%, von 7% bis 1%, von 7% bis 1,5%, von 7% bis 2%, von 7% bis 2,5%, von 7% bis 3%, von 7% bis 3,5%, von 7% bis 4%, von 6% bis 0,1%, von 6% bis 0,5%, von 6% bis 1%, von 6% bis 1,5%, von 6% bis 2%, von 6% bis 2,5%, von 6% bis 3%, von 6% bis 3,5%, von 6% bis 4%, von 5% bis 0,1%, von 5% bis 0,5% oder von 5% bis 1%.

**3.** Mischung nach Anspruch 1 oder 2, wobei:

- der Molprozentanteil an Lithiumbis(fluorsulfonyl)imid größer oder gleich 95 % ist und
- der Molprozentanteil an Lithium-2-trifluormethyl-4,5-dicyanoimidazolat kleiner oder gleich 5 % ist.

**4.** Mischung nach Anspruch 1 oder 2, umfassend:

- 86 bis 99,9 Mol-%, bevorzugt 90 bis 99,5 Mol-%, im Besonderen 92 bis 98 Mol-% und vorzugsweise 93 bis 97 Mol-%, beispielsweise 95 Mol-%, Lithiumbis-(fluorsulfonyl)imid und
- 14 bis 0,1 Mol-%, bevorzugt 10 bis 0,5 Mol-%, im Besonderen 8 bis 2 Mol-% und vorzugsweise 7 bis 3 Mol-%, beispielsweise 5 Mol-%, Lithium-2-trifluormethyl-4,5-dicyanoimidazolat.

**5.** Verwendung einer Mischung nach einem der Ansprüche 1 bis 4 in einer Li-Ionen-Batterie, im Besonderen in einem Temperaturbereich zwischen -30 °C und 65 °C, vorzugsweise zwischen -25 °C und 60 °C, bevorzugt bei einer Temperatur größer oder gleich 25 °C, bevorzugt zwischen 25 °C und 65 °C, vorteilhafterweise zwischen 40 °C und 60 °C.

**6.** Elektrolytzusammensetzung, umfassend die Mischung von Lithiumsalzen nach einem der Ansprüche 1 bis 4, mindestens ein Lösungsmittel und gegebenenfalls ein Elektrolytadditiv.

**7.** Zusammensetzung nach Anspruch 6, wobei die molare Konzentration von Lithiumbis(fluorsulfonyl)imid und Lithium-2-trifluormethyl-4,5-dicyanoimidazolat in der Elektrolytzusammensetzung kleiner oder gleich 5 mol/l, vorteilhafterweise kleiner oder gleich 4 mol/l, bevorzugt kleiner oder gleich 2 mol/l, vorzugsweise kleiner oder gleich 1,5 mol/l und im Besonderen kleiner oder gleich 1 mol/l ist.

**8.** Zusammensetzung nach Anspruch 6 oder 7, umfassend:

- 0,85 bis 0,999 mol/l Lithiumbis(fluorsulfonyl)-imid und
- 0,15 bis 0,001 mol/l Lithium-2-trifluormethyl-4,5-dicyanoimidazolat.

**9.** Zusammensetzung nach einem der Ansprüche 6 bis 8, umfassend:

- 0,86 bis 0,999 mol/l, bevorzugt 0,86 bis 0,99 mol/l, insbesondere 0,90 bis 0,995 mol/l, im Besonderen 0,92 bis 0,98 mol/l und vorzugsweise 0,93 bis 0,97 mol/l, beispielsweise 0,95 mol/l, Lithiumbis(fluorsulfonyl)imid und
- 0,14 bis 0,001 mol/l, bevorzugt 0,14 bis 0,01 mol/l, insbesondere 0,10 bis 0,005 mol/l, im Besonderen 0,08 bis 0,2 mol/l und vorzugsweise 0,07 bis 0,03 mol/l, beispielsweise 0,05 mol/l, Lithium-2-trifluormethyl-4,5-dicyanoimidazolat.

**10.** Zusammensetzung nach einem der Ansprüche 6 bis 9, wobei das Lösungsmittel aus der Gruppe bestehend aus

Ethern, Carbonaten, Estern, Ketonen, teilhydrierten Kohlenwasserstoffen, Nitrilen, Amiden, Alkoholen, Sulfoxiden, Sulfolan, Nitromethan, 1,3-Dimethyl-2-imidazolidinon, 1,3-Dimethyl-3,4,5,6-tetrahydro-2(1H)-pyrimidinon, 3-Methyl-2-oxazolidinon und Mischungen davon ausgewählt ist.

**11.** Zusammensetzung nach einem der Ansprüche 6 bis 10, wobei das Lösungsmittel aus Carbonaten und Mischungen davon ausgewählt ist, beispielsweise aus den folgenden Mischungen:

- Ethylencarbonat/Propylencarbonat/Dimethylcarbonat in einem Massenverhältnis von 1/1/1;
- Ethylencarbonat/Propylencarbonat/Diethylcarbonat in einem Massenverhältnis von 1/1/1;
- Ethylencarbonat/Propylencarbonat/Methylethylcarbonat in einem Massenverhältnis von 1/1/1;
- Ethylencarbonat/Dimethylcarbonat in einem Massenverhältnis von 1/1;
- Ethylencarbonat/Diethylcarbonat in einem Massenverhältnis von 1/1;
- Ethylencarbonat/Methylethylcarbonat in einem Massenverhältnis von 1/1;
- Ethylencarbonat/Dimethylcarbonat in einem Volumenverhältnis von 3/7;
- Ethylencarbonat/Diethylcarbonat in einem Volumenverhältnis von 3/7;
- Ethylencarbonat/Methylethylcarbonat in einem Volumenverhältnis von 3/7.

**12.** Zusammensetzung nach einem der Ansprüche 6 bis 11, wobei das Elektrolytadditiv aus der Gruppe bestehend aus Fluorethylencarbonat, Vinylencarbonat, 4-Vinyl-1,3-dioxolan-2-on, Pyridazin, Vinylpyridazin, Chinolin, Vinylchinolin, Butadien, Sebaconitril, $LiB(C_2O_4)_2$, Lithiumnitrat, Alkyldisulfiden, Fluortoluol, 1,4-Dimethoxytetrafluortoluol, Oximen, aliphatischen Epoxiden, halogenierten Biphenylen, Methacrylsäuren, Allylethylcarbonat, Vinylacetat, Divinyladipat, Acrylnitril, 2-Vinylpyridin, Maleinsäureanhydrid, Methylcinnamat, Phosphonaten, Silanverbindungen, die ein Vinyl enthalten, 2-Cyanofuran und Mischungen davon ausgewählt ist, wobei es sich bei dem Elektrolytadditiv bevorzugt um Fluorethylencarbonat handelt.

**13.** Verwendung einer Elektrolytzusammensetzung nach einem der Ansprüche 6 bis 12 in einer Li-Ionen-Batterie, im Besonderen in einem Temperaturbereich zwischen -30 °C und 65 °C, vorzugsweise zwischen - 25 °C und 60 °C, bevorzugt bei einer Temperatur größer oder gleich 25 °C, bevorzugt zwischen 25 °C und 65 °C, vorteilhafterweise zwischen 40 °C und 60 °C.

**14.** Elektrochemische Zelle mit einer negativen Elektrode, einer positiven Elektrode und einer Elektrolytzusammensetzung nach einem der Ansprüche 6 bis 12, welche zwischen der negativen Elektrode und der positiven Elektrode angeordnet ist.

**15.** Batterie mit mindestens einer elektrochemischen Zelle nach Anspruch 14.

## Claims

**1.** A mixture of lithium salts comprising:

- from 85 mol% to 99.9 mol% of lithium bis(fluorosulfonyl)imide; and
- from 0.1 mol% to 15 mol% of lithium 2-trifluoromethyl-4,5-dicyanoimidazolate.

**2.** The mixture as claimed in claim 1, comprising:

- lithium bis(fluorosulfonyl)imide in one of the following molar percentages: from 85% to 99%, from 85% to 98.5%, from 85% to 98%, from 85% to 97.5%, from 85% to 97%, from 85.5% to 99.9%, from 86% to 99.9%, from 86% to 99.5%, from 86.5% to 99.5%, from 87% to 99.5%, from 87% to 99%, from 87.5% to 99.9%, from 87.5% to 99.5%, from 87.5% to 99%, from 88% to 99.9%, from 88% to 99.5%, from 88% to 99%, from 89% to 99.9%, from 89% to 99.5%, from 89% to 99%, from 89.5% to 99.9%, from 89.5% to 99.5%, from 89.5% to 99%, from 90% to 99.9%, from 90% to 99.5%, from 90% to 99.5%, from 90% to 99%, from 90% to 98.5%, from 90% to 98%, from 90% to 97.5%, from 90% to 97%, from 90% to 96.5%, from 90% to 96%, from 91% to 99.9%, from 91% to 99.5%, from 91% to 99%, from 91% to 98.5%, from 91% to 98%, from 91% to 97.5%, from 91% to 97%, from 91% to 96.5%, from 91% to 96%, from 92% to 99.9%, from 92% to 99.5%, from 92% to 99%, from 92% to 98.5%, from 92% to 98%, from 92% to 97.5%, from 92% to 97%, from 92% to 96.5%, from 92% to 96%, from 93% to 99.9%, from 93% to 99.5%, from 93% to 99%, from 93% to 98.5%, from 93% to 98%, from 93% to 97.5%, from 93% to 97%, from 93% to 96.5%, from 93% to 96%, from 94% to 99.9%, from 94% to 99.5%, from

94% to 99%, from 94% to 98.5%, from 94% to 98%, from 94% to 97.5%, from 94% to 97%, from 94% to 96.5%, from 94% to 96%, from 95% to 99.9%, from 95% to 99.5%, or from 95% to 99%; and
- lithium 2-trifluoromethyl-4,5-dicyanoimidazolate in one of the following molar percentages: from 15% to 1%, from 15% to 1.5%, from 15% to 2%, from 15% to 2.5%, from 15% to 3%, from 14.5% to 0.1%, from 14% to 0.1%, from 14% to 0.5%, from 13.5% to 0.5%, from 13% to 0.5%, from 13% to 1%, from 12.5% to 0.1%, from 12.5% to 0.5%, from 12.5% to 1%, from 12% to 0.1%, from 12% to 0.5%, from 12% to 1%, from 11% to 0.1%, from 11% to 0.5%, from 11% to 1%, from 10.5% to 0.1%, from 10.5% to 0.5%, from 11.5% to 1%, from 10% to 0.1%, from 10% to 0.5%, from 10% to 1%, from 10% to 1.5%, from 10% to 2%, from 10% to 2.5%, from 10% to 3%, from 10% to 3.5%, from 10% to 4%, from 9% to 0.1%, from 9% to 0.5%, from 9% to 1%, from 9% to 1.5%, from 9% to 2%, from 9% to 2.5%, from 9% to 3%, from 9% to 3.5%, from 9% to 4%, from 8% to 0.1%, from 9% to 0.5%, from 8% to 1%, from 8% to 1.5%, from 8% to 3%, from 8% to 2.5%, from 8% to 3%, from 8% to 3.5%, from 8% to 4%, from 7% to 0.1%, from 7% to 0.5%, from 7% to 1%, from 7% to 1.5%, from 7% to 2%, from 7% to 2.5%, from 7% to 3%, from 7% to 3.5%, from 7% to 4%, from 6% to 0.1%, from 6% to 0.5%, from 6% to 1%, from 6% to 1.5%, from 6% to 2%, from 6% to 2.5%, from 6% to 3%, from 6% to 3.5%, from 6% to 4%, from 5% to 0.1%, from 5% to 0.5%, or from 5% to 1%.

**3.** The mixture as claimed in either one of claims 1 and 2, in which:

- the molar percentage of lithium bis(fluorosulfonyl)imide is greater than or equal to 95%; and
- the molar percentage of lithium 2-trifluoromethyl-4,5-dicyanoimidazolate is less than or equal to 5%.

**4.** The mixture as claimed in either one of claims 1 and 2, comprising:

- from 86 mol% to 99.9 mol%, preferably from 90 mol% to 99.5 mol%, in particular from 92 mol% to 98 mol% and preferentially from 93 mol% to 97 mol%, for example 95 mol%, of lithium bis(fluorosulfonyl)imide; and
- from 14 mol% to 0.1 mol%, preferably from 10 mol% to 0.5 mol%, in particular from 8 mol% to 2 mol% and preferentially from 7 mol% to 3 mol%, for example 5 mol%, of lithium 2-trifluoromethyl-4,5-dicyanoimidazolate.

**5.** The use of a mixture as claimed in any one of claims 1 to 4, in a Li-ion battery, in particular in a temperature range of between -30°C and 65°C, preferentially between -25°C and 60°C, preferably at a temperature of greater than or equal to 25°C, preferably of between 25°C and 65°C, advantageously between 40°C and 60°C.

**6.** An electrolyte composition, comprising the mixture of lithium salts as claimed in any one of claims 1 to 4, at least one solvent and optionally at least one electrolytic additive.

**7.** The composition as claimed in claim 6, in which the molar concentration of lithium bis(fluorosulfonyl)imide and lithium 2-trifluoromethyl-4,5-dicyanoimidazolate in the electrolyte composition is less than or equal to 5 mol/l, advantageously less than or equal to 4 mol/l, preferably less than or equal to 2 mol/l, preferentially less than or equal to 1.5 mol/l and in particular less than or equal to 1 mol/l.

**8.** The composition as claimed in either one of claims 6 and 7, comprising:

- from 0.85 to 0.999 mol/l of lithium bis(fluorosulfonyl)imide; and
- from 0.15 to 0.001 mol/l of lithium 2-trifluoromethyl-4,5-dicyanoimidazolate.

**9.** The composition as claimed in any one of claims 6 to 8, comprising:

- from 0.86 to 0.999 mol/l, preferably from 0.86 to 0.99 mol/l, in particular from 0.90 to 0.995 mol/l, especially from 0.92 to 0.98 mol/l and preferentially from 0.93 to 0.97 mol/l, for example 0.95 mol/l, of lithium bis(fluorosulfonyl)imide; and
- from 0.14 to 0.001 mol/l, preferably from 0.14 to 0.01 mol/l, in particular from 0.10 to 0.005 mol/l, especially from 0.08 to 0.2 mol/l and preferentially from 0.07 to 0.03 mol/l, for example 0.05 mol/l, of lithium 2-trifluoromethyl-4,5-dicyanoimidazolate.

**10.** The composition as claimed in any one of claims 6 to 9, in which the solvent is chosen from the group consisting of ethers, carbonates, esters, ketones, partially hydrogenated hydrocarbons, nitriles, amides, alcohols, sulfoxides, sulfolane, nitromethane, 1,3-dimethyl-2-imidazolidinone, 1,3-dimethyl-3,4,5,6-tetrahydro-2(1H)-pyrimidinone, 3-methyl-2-oxazolidinone and of their mixtures.

**11.** The composition as claimed in any one of claims 6 to 10, in which the solvent is chosen from carbonates and their mixtures, for example from the following mixtures:

- ethylene carbonate/propylene carbonate/dimethyl carbonate in a 1/1/1 ratio by weight;
- ethylene carbonate/propylene carbonate/diethyl carbonate in a 1/1/1 ratio by weight;
- ethylene carbonate/propylene carbonate/ethyl methyl carbonate in a 1/1/1 ratio by weight;
- ethylene carbonate/dimethyl carbonate in a 1/1 ratio by weight;
- ethylene carbonate/diethyl carbonate in a 1/1 ratio by weight;
- ethylene carbonate/ethyl methyl carbonate in a 1/1 ratio by weight;
- ethylene carbonate/dimethyl carbonate in a 3/7 ratio by volume;
- ethylene carbonate/diethyl carbonate in a 3/7 ratio by volume;
- ethylene carbonate/ethyl methyl carbonate in a 3/7 ratio by volume.

**12.** The composition as claimed in any one of claims 6 to 11, in which the electrolytic additive is chosen from the group consisting of fluoroethylene carbonate, vinylene carbonate, 4-vinyl-1,3-dioxolan-2-one, pyridazine, vinylpyridazine, quinoline, vinylquinoline, butadiene, sebaconitrile, $LiB(C_2O_4)_2$, lithium nitrate, alkyl disulfides, fluorotoluene, 1,4-dimethoxytetrafluorotoluene, oximes, aliphatic epoxides, halogenated biphenyls, methacrylic acids, allyl ethyl carbonate, vinyl acetate, divinyl adipate, acrylonitrile, 2-vinylpyridine, maleic anhydride, methyl cinnamate, phosphonates, silane compounds containing a vinyl, 2-cyanofuran and of their mixtures, the electrolytic additive preferably being fluoroethylene carbonate.

**13.** The use of an electrolyte composition as claimed in any one of claims 6 to 12, in a Li-ion battery, in particular in a temperature range of between -30°C and 65°C, preferentially between -25°C and 60°C, preferably at a temperature of greater than or equal to 25°C, preferably of between 25°C and 65°C, advantageously between 40°C and 60°C.

**14.** An electrochemical cell comprising a negative electrode, a positive electrode and an electrolyte composition as claimed in any one of claims 6 to 12, interposed between the negative electrode and the positive electrode.

**15.** A battery comprising at least one electrochemical cell as claimed in claim 14.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2983466
- WO 2015158979 A
- WO 2013072591 A